# EUROPEAN PATENT APPLICATION

(11) **EP 4 566 594 A1**
(43) Date of publication of application: **11.06.2025**
(21) Application number: 23850481.5
(22) Date of filing: 04.08.2023
(51) Int. Cl.: A61K 9/51, A61K 31/164, A61P 35/00

(54) **LIPID NANOPARTICLES COMPRISING LONG-CHAIN CERAMIDE AND COMPOSITION FOR CELL DEATH COMPRISING SAME**

(30) Priority: 05.08.2022 KR 20220097752
(71) Applicant: Croda Korea Limited, Iksan-si, Jeollabuk-do 54588 (KR)
(72) Inventor: YOON, Tae Jong, Seoul 05849 (KR); WON, Eun Jeong, Incheon 21535 (KR); PARK, Seung Won, Yongin-si, Gyeonggi-do 16894 (KR); KIM, Sang Chul, Yongin-si, Gyeonggi-do 17095 (KR); KIM, A Ra, Yongin-si, Gyeonggi-do 16838 (KR)
(74) Representative: de Saint Viance, Isabelle Marie Fanny
(86) International application number: PCT/KR2023/011464
(87) International publication number: WO 2024/029981

(57) **Abstract**

A long-chain ceramide such as an exogenous C18-ceramide has very low solubility in an aqueous solution and thus has limited use, and when even used as a drug, has had poor cell delivery. In this regard, the prevent invention provides lipid nanoparticles comprising a long-chain ceramide and a preparation method therefor. Through the present invention, by delivering long-chain ceramides such as C18-ceramides to an in vivo target through lipid nanoparticles, cell death may be effectively induced according to the purpose, and utilization as a cell therapy product with high water solubility and high water dispersibility may be increased.

## Description

### Field of the Invention

The present invention relates to lipid nanoparticles comprising a long-chain ceramide, and to a nanoparticle delivery system composition for cell apoptosis having excellent water solubility and water dispersibility comprising the same.

The present invention also relates to a method for preparing lipid nanoparticles having high *in vivo* target delivery properties comprising long-chain ceramide, and to a method for inducing cell apoptosis using the nanoparticles.

### Background

Ceramides are sphingolipids composed of sphingosine and fatty acids of various chain lengths. The endogenous form of ceramides is usually composed of long carbon chain lengths ranging from C16 to C24 in animal cells. Six types of ceramide synthases exist in animal cells, which are responsible for attaching fatty acyl-CoA of specific lengths to long-chain bases to determine the acyl chain length of sphingolipids, including ceramides, sphingomyelins, and glycosphingolipids.

C16 to C24-ceramides, which are divided according to the carbon chain length, can act as active signaling molecules that regulate various cellular processes. In particular, C18-ceramide can contribute to cell death and cell proliferation inhibition by being catalyzed by ceramide synthase 1 and 4 in the *de novo* synthesis pathway. However, since these long-chain ceramides have extremely low solubility in aqueous solutions, their use is extremely limited despite their efficacy when they can only be dissolved in organic solvents such as ethanol. In addition, since C16 to C20-ceramides mostly exhibit anti-proliferative and apoptotic effects, if they are not delivered to specific cells such as cancer cells, they can cause side effects such as cell death of normal cells. In this regard, there is an urgent need for a method that can disperse and utilize long-chain ceramides with extremely low water solubility.

### [Prior Art Literature]

### [Patent Document]

(Patent Document 1) Republic of Korea Registered Patent No. 10-2325570 (Cosmetic composition for moisturizing and improving skin barrier containing nanoliposomes including oxygen water and ceramide and method for preparing the same)

(Patent Document 2) Republic of Korea Patent No. 10-0713555 (Hair protection composition containing nanoliposomes stabilized by encapsulating ceramide as an effective ingredient)

(Patent Document 3) Republic of Korea Registered Patent No. 10-2267378 (Pharmaceutical composition for preventing or treating breast cancer containing C12, C16 or C18-ceramide as an active ingredient)

(Patent Document 4) Republic of Korea Registered Patent No. 10-1796036 (Nanoliposome delivery carrier composition encapsulating a complex of Cas9 protein, guide RNA that inhibits the expression of KRAS gene, and cationic polymer, or a treatment agent for colorectal cancer resistant to anticancer drugs due to KRAS gene mutation containing the same)

(Patent Document 5) Republic of Korea Registered Patent No. 10-1710026 (Nanoliposome delivery carrier composition containing hybrid of Cas9 protein and guide RNA)

(Patent Document 6) Republic of Korea Registered Patent No. 10-2328197 (Nanoliposome delivery carrier composition having editing function of KRAS and p53 genes)

(Patent Document 7) Japanese Patent No. 6944061 (Nanoliposome-microbubble complex encapsulating a complex of Cas9 protein, guide RNA that suppresses the expression of SRD5A2 gene, and cationic polymer, and a composition containing the same for treating or preventing hair loss)

(Patent Document 8) Japanese Registered Patent No. 6876202 (Nanoliposome-microbubble complex encapsulated with a drug for hair loss treatment and composition containing the same for alleviating or treating hair loss)

(Patent Document 9) Japanese Registered Patent No. 3920330 (Polyethylene glycol modified ceramide lipids and their use in liposomes)

### Detailed description of the invention

### Technical problem

The purpose of the present invention is to provide lipid nanoparticles including long-chain ceramide having excellent water solubility and water dispersibility by manufacturing a lipid nanoparticle (LNP) composition by incorporating exogenous long-chain ceramides into a lipid layer.

In addition, the present invention provides an excellent cell apoptosis composition comprising lipid nanoparticles containing long-chain ceramides having excellent water solubility and water dispersibility.

Further, the present invention provides an anticancer composition comprising lipid nanoparticles containing long-chain ceramides having excellent water solubility and water dispersibility, which have high target delivery properties in the body.

### Problem solving means

The present invention relates to lipid nanoparticles comprising a long-chain ceramide having the structure represented by the following Chemical Formula 1.

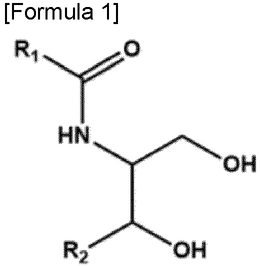

(In the above Chemical Formula 1, R₁ is a saturated or unsaturated aliphatic chain having 17 carbon atoms, R₂ is one selected from phytosphingosine, sphingosine, and sphinganine, and has 10 to 16 carbon atoms)

The long-chain ceramide having the structure of the above Chemical Formula 1 may be a compound selected from a N-oleoyl-phytosphingosine-based compound or a derivative thereof; a N-stearoyl-erythro-sphingosine-based compound or a derivative thereof; a N-oleoyl-erythro-sphingosine-based compound or a derivative thereof; a N-oleoyl-erythro-sphingosine-based compound or a derivative thereof; a N-stearoyl-erythro-sphinganine-based compound or a derivative thereof; and a N-oleoyl-erythro-sphinganine-based compound or a derivative thereof.

The N-oleoyl-phytosphingosine-based compounds or derivative thereof may be a compound of the following Chemical Formula 2 or a derivative thereof.

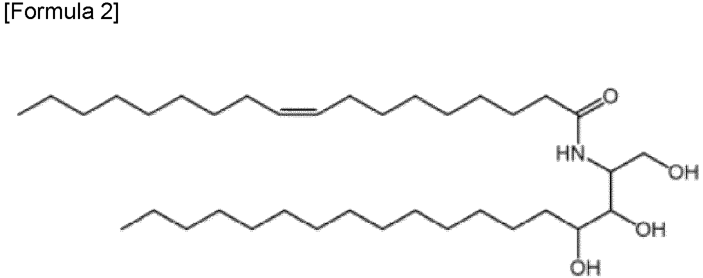

The above N-stearoyl-erythro-sphingosine-based compounds or derivative thereof may be a compound selected from the following Chemical Formulas 3 and 4 or a derivative thereof.

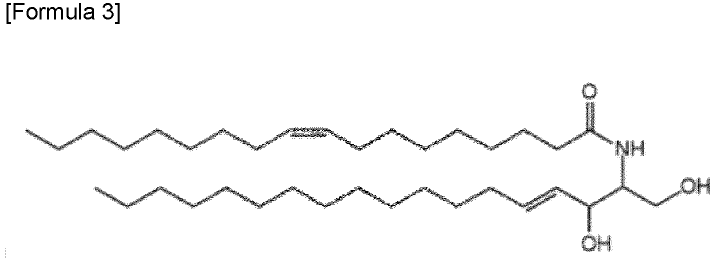

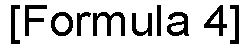

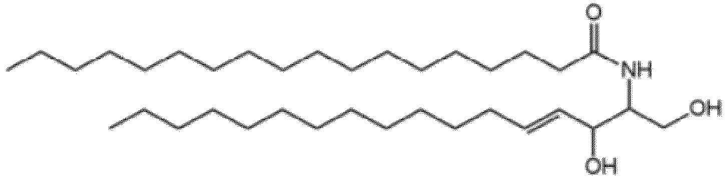

The N-oleoyl-erythro-sphingosine-based compounds or derivative thereof may be a compound selected from the following Chemical Formulas 5 and 6, or a derivative thereof.

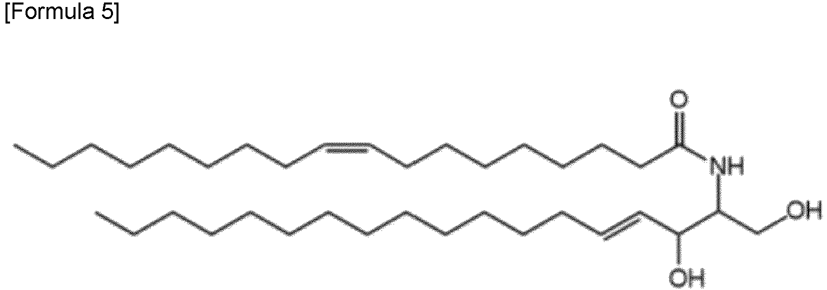

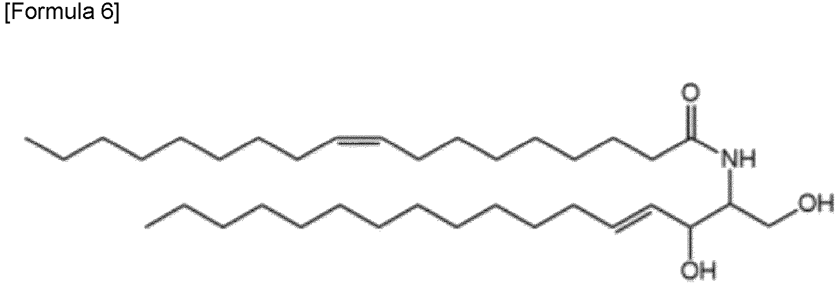

The N-stearoyl-erythro-sphinganine-based compound or derivative thereof may be a compound of the following Chemical Formula 7 or a derivative thereof.

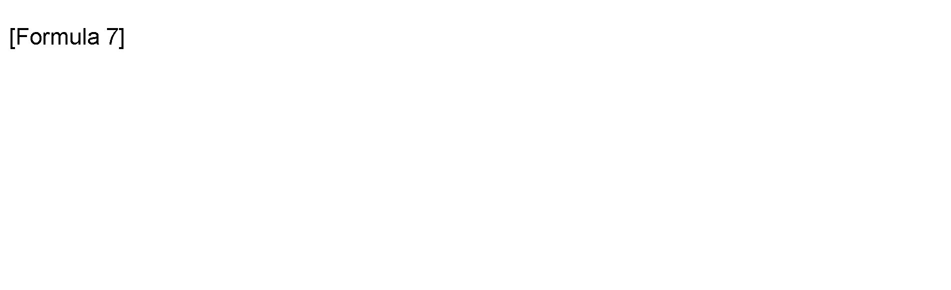

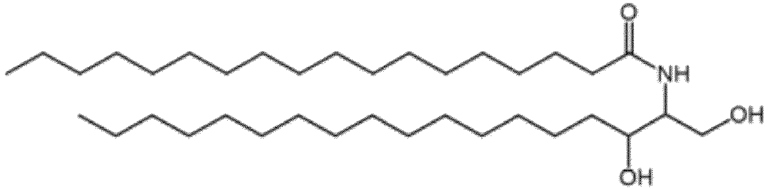

The N-oleoyl-erythro-sphinganine-based compound or derivative thereof may be a compound of Chemical Formula 8 or a derivative thereof.

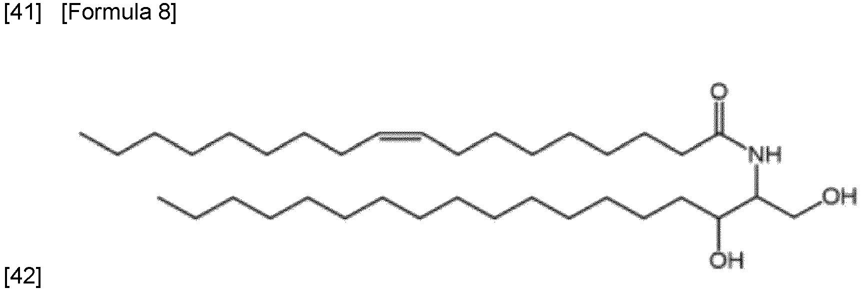

The lipid nanoparticle is characterized by including a long-chain ceramide of Chemical Formula 1, and

The composition is characterized by comprising at least one lipid selected from lecithin; cholesterol; a non-cationic lipid; and a PEG-modified lipid. Preferably, it may include all of the lecithin, cholesterol, non-cationic lipid, and PEG-modified lipid.

The lecithin is widely distributed in the animal/plant kingdom and thus has excellent biocompatibility, and its stability has already been verified, and thus it is widely used in food and pharmaceutical delivery technology. In addition, it can be used as a material that facilitates size control and modification of lipid nanoparticles.

The cholesterol provides morphological rigidity to lipid charging in the lipid nanoparticle, and plays a role in improving the stability of the nanoparticle by being dispersed in the core and surface of the nanoparticle.

The non-cationic lipid may include at least one selected from neutral lipid and anionic lipid.

The neutral lipid refers to a lipid that is uncharged or has a neutral zwitterion form within a pH range of 4.0 to 8.0. In addition, the neutral lipid may include any neutral lipid known to those skilled in the art. For example, the neutral lipid may be HSPC (hydrogenated soy phosphatidylcholine), DOPC (1, 2-dioleoyl-*sn*-glycero-3-phosphocholine), POPE (1-palmitoyl-2-oleoyl-*sn*-glycero-3-phosphoethanolamine), DSPC (1, 2-distearoyl-*sn*-glycero-3-phosphocholine), DSPE (1, 2-distearoyl-*sn*-glycero-3-phosphoethanolamine), DPPC (1, 2-dipalmitoyl-*sn*-glycero-3-phosphocholine), POPC (1-palmitoyl-2-oleoyl-*sn*-glycero-3-phosphocholine), DOPE (1, 2-dioleoyl-*sn*-glycero-3-phosphoethanolamine), SM (N-palmitoyl-D-erythro-spinolphosphorylcholine), DLPE (1, 2-dilauroyl-*sn*-glycero-3-phosphoethanolamine), DPPE (1, 2-diphytanoyl-*sn*-glycero-3-phosphoethanolamine), phosphatidylcholine, dimyristoylglycerol, succinoyl-diacylglycerol, phosphatidyl ethanolamine, tetraether lipid, sphingolipid, diacryl glycerol and glyceride, or a combination thereof.

The anionic lipid refers to any amphiphilic lipid having at least one negative charge within the range of pH 4.0 to pH 8.0. In addition, the anionic lipid may include any anionic lipid known to those skilled in the art. For example, the anionic lipid may be DOPS (dioleoylphosphatidylserine), DMPG (dimyristoylphosphatidylglycerol), DPPG (dipalmitoylphosphatidylglycerol), DTPA (diethylenetriamine pentaacetic acid), DPTGA (1,4-dipalmitoyl-tartarate-2,3-diglutaric acid), DSTSA (1,4-disteroyl-tartarate-2,3-disuccinic acid), CHHDA (2-carboxyheptadecanoyl heptadecylamide), DMPS (dimyristoylphosphatidylserine), DPPS (dipalmitoylphosphatidylserine), POPS (dioleoylphosphatidylglycerol), DOPG (palmitoylphosphatidylglycerol), POPG (dimyristoylphosphatidylphosphatidic acid), DMPA (dipalmitoylphosphatidic acid), DPPA (dithiophosphoric acid), DOPA (dithiophosphoric acid), POPA (dithioaminothiophosphoric acid), CetylP (Cetyl phosphate), and CHEMS (cholesterol hemisuccinate), or a combination thereof.

The lipid of the PEG-modified lipid can be used without limitation as long as it is a lipid that can bind to polyethylene glycol. Preferably, it can be a non-cationic phospholipid that can bind to polyethylene glycol (PEG).

The PEG in the above PEG-modified lipid is a hydrophilic polymer that has the ability to inhibit the adsorption of plasma proteins, thereby increasing the circulation time of lipid nanoparticles in the body and preventing aggregation between nanoparticles. In addition, PEG-modified lipid exhibits a stealth function *in vivo,* thereby preventing the degradation of nanoparticles.

The above PEG may be a functionalized PEG in which a functional group is bonded to a side that is not bonded to the lipid. In this case, the usable functional group may be one or more selected from the group consisting of a succinyl group, a carboxylic acid group, a maleimide group, an amine group, a biotin group, a cyanur group, a folate group, and the like.

The above PEG refers to a typical polyethylene glycol, and in the present invention, POZ [poly(2-oxazoline)] can be used as a substitute for it.

The lipid nanoparticle may have a particle size of 10 to 2,000 nm. If the size of the lipid nanoparticle is less than 10 nm or more than 2,000 nm, the particle stability may be reduced, which is not preferable.

The lipid nanoparticles are preferably contained in a ratio of 2 to 5 mol of the long-chain ceramide having the structure of Chemical Formula 1, 0.5 to 5 mol of lecithin, 0.1 to 1mol of cholesterol, 0.01 to 0.5 mol of a non-cationic lipid (such as DSPC), and 0.05 to 0.5 mol of a PEG-modified lipid (such as DSPE-PEG(2000) amine).

The present invention relates to a nanoparticle delivery composition for cell apoptosis, comprising lipid nanoparticles including a long-chain ceramide represented by the above Formula 1.

The composition may be a composition containing 1 x 10⁹ to 1 x 10¹² particles per mL.

The cell may be a cancer cell. The cancer cell may be derived from a cancer tissue selected from angiomas, vascular fibromas, lung cancer, non-small cell lung cancer, liver cancer, colon cancer, bone cancer, pancreatic cancer, skin cancer, head cancer, cervical cancer, melanoma, uterine cancer, ovarian cancer, rectal cancer, stomach cancer, anal cancer, colon cancer, breast cancer, fallopian tube carcinoma, endometrial carcinoma, cervical carcinoma, vaginal carcinoma, vulvar carcinoma, Hodgkin's disease, oesophageal cancer, small intestine cancer, endocrine gland cancer, thyroid cancer, parathyroid cancer, adrenal cancer, soft tissue sarcoma, urethral cancer, penile cancer, prostate cancer, bladder cancer, kidney or ureter cancer, kidney cell carcinoma, kidney pelvic carcinoma, central nervous system tumor, primary central nervous system lymphoma, spinal cord tumor, brainstem glioma, and pituitary adenoma.

The present invention also relates to a method for producing a lipid nanoparticle comprising the long-chain ceramide of Chemical Formula 1.

Preferably, (step 1) a step of mixing at least one lipid selected from neutral lipid; cholesterol; non-cationic lipid; and PEG-modified lipid; and long-chain ceramide of Chemical Formula 1 in an organic solvent to prepare a mixed composition;
(step 2) A step of producing the mixed composition in a dispersion state and purifying it by a column; and
(step 3) A step of sterilizing and filtering the purified product obtained by column purification;
may be included.

In the step 1, the concentration of the long-chain ceramide having the structure of the Chemical Formula 1 is preferably 1 to 5 mol, more preferably 2 to 5 mol. In this case, if the concentration of long-chain ceramide exceeds 5 mol, the size of the final nanoparticles manufactured is not uniform or the number of particles manufactured is reduced, making them unsuitable for functioning as a drug delivery vehicle.

The above lipid may include lecithin, cholesterol, non-cationic lipid, and PEG-modified lipid. Preferably, it includes 0.1 to 10 mol lecithin, 0.01 to 1 mol cholesterol, 0.005 to 1 mol non-cationic lipid, and 0.01 to 1 mol PEG-modified lipid, and more preferably, it includes 0.5 to 5 mol lecithin, 0.1 to 1 mol cholesterol, 0.01 to 0.5 mol non-cationic lipid, and 0.05 to 0.5 mol PEG-modified lipid.

The organic solvent may be selected from methanol, ethanol, benzene, butanol, butyl acetate, carbon tetrachloride, chloroform, cyclohexane, dichloroethane, dichloromethane, diethyl ether, diisopropyl ether, ethyl acetate, heptane, hexane, isooctane, methyl ethyl ketone (MEK), methyl t-butyl ether (MTBE), pentane, toluene, trichloroethylene, xylene, and a mixed solvent thereof.

In this case, in the step 2, a dispersion may be prepared by sonication or freeze-thawing. When the process of freezing or thawing the mixed composition is performed, it may be preferably repeated 1 to 12 times. By repeating the freezing and thawing steps of the mixed composition, lipid nanoparticles having a more uniform size may be formed, and the formation efficiency of the lipid nanoparticles may be increased. If it exceeds 12 times, the formation of lipid nanoparticles of a uniform size may rather decrease, so it is preferable to be within 12 times.

In this case, in the step 2, the dispersion may be prepared in a separate reactor. Lipid nanoparticles may be formed by repeating the process of freezing or thawing the mixed composition in the reactor.

As another method, in the step 2, a dispersion may be prepared with a droplet-based microfluidic system.

The filter used for the sterile filtration of step 3 is characterized in that it has a pore size of 0.25 to 0.4 µm.

The composition of the present invention may be provided as a pharmaceutical composition or a cosmetic composition.

The pharmaceutical composition may be formulated and used in the form of oral formulations such as powders, granules, tablets, capsules, suspensions, emulsions, syrups, aerosols, etc., external preparations, suppositories, and sterile injectable solutions, respectively, according to conventional methods. The carrier, excipient and diluent that may be included in the pharmaceutical composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. In the case of formulation, it is prepared by using a diluent or excipient such as a filler, an extender, a binder, a wetting agent, a disintegrant, a surfactant, etc. Solid formulations for oral administration include tablets, pills, powders, granules, capsules, and the like, and such solid formulations are prepared by mixing the composition of the present invention with at least one excipient, for example, starch, calcium carbonate, sucrose or lactose, gelatin, and the like. In addition to simple excipients, lubricants such as magnesium stearate and talc are also used. Liquid formulations for oral administration include suspensions, solutions for internal use, emulsions, syrups, and the like, and may contain various excipients, for example, wetting agents, sweeteners, fragrances, preservatives, and the like, in addition to commonly used simple diluents such as water and liquid paraffin. Formulations for parenteral administration include sterile aqueous solutions, non-aqueous solvents, suspensions, emulsions, lyophilized formulations, and suppositories. As the non-aqueous solvent and the suspending agent, propylene glycol, polyethylene glycol, vegetable oil such as olive oil, injectable ester such as ethyl oleate, and the like may be used. As a base for suppositories, witepsol, macrogol, tween 61, cacao butter, laurin butter, glycerogelatin, etc. may be used.

The dosage of the pharmaceutical composition of the present invention will vary depending on the age, sex, and weight of the subject to be treated, the specific disease or pathological condition to be treated, the severity of the disease or pathological state, the route of administration, and the judgment of the prescriber. The determination of the dosage based on these factors is within the level of those skilled in the art, and the dosage is generally in the range of 0.01 mg/kg/day to about 2000 mg/kg/day. A more preferred dosage is 1 mg/kg/day to 500 mg/kg/day. The administration may be administered once a day or divided into several times. The above dosage does not limit the scope of the present invention in any way.

The pharmaceutical composition of the present invention may be administered to mammals such as rats, livestock, and humans *via* various routes. All modes of administration can be envisaged, for example, by oral, rectal, or intravenous, intramuscular, subcutaneous, intrauterine, or intracerebroventricular injection.

The formulation of the cosmetic composition of the present invention may be prepared in any formulation commonly prepared in the art, and may be selected from essence, lotion, emulsion, pack, hand cream, foot cream, lip balm, lipstick, eyeshadow, eyeliner, eyebrow pencil, blusher, highlighter, general lotion, skin, cream, serum, beauty soap, softening lotion, medicinal lotion, whole body detergent, cleansing foam, cleansing lotion, gel, cleansing oil, cleansing cream, shampoo, rinse, hair treatment, hair lotion, cleansing tissue, and cleansing water.

The cosmetic composition may include any suitable excipient composition. Preferably, they may include oil and fat components, emollient agents, surfactants, organic and inorganic pigments, organic powders, ultraviolet absorbers, preservatives, bactericides, antioxidants, pH adjusting agents, alcohols, colour agents, flavouring agents, blood circulation promoters, cold reducing agents, limiters, purified water, etc.

In addition, the cosmetic composition may contain another active ingredient, and preferably, may contain an anti-wrinkle agent, a skin aging inhibitor, a skin whitening agent, an antioxidant, a skin anti-inflammatory agent, a moisturizer, or a hair growth agent.

As the above-mentioned wrinkle-improving agent, substances that inhibit MMP-1, an extracellular matrix (ECM) protein-decomposing enzyme, can be selected, such as silicic acid, N-methyl-L-serine, isoflavonoids, dehydroepiandrosterone, and paeoniflorin, substances that prevent skin aging by removing active oxygen that promotes the collapse of ECM, benzastatins and coenzyme Q10, and the like, can be used. In addition, adenosine, ascorbyl glucoside, kinetin, auxin, peptide, retinol, retinyl palmitate, polyethoxylated retinamide, alpha hydroxyl acid, etc., which are known to have various wrinkle-improving effects, can be used.

The whitening agent may include arbutin, niacinamide, ascorbic acid, magnesium ascorbyl phosphate, ascorbyl acid-2-glucoside, paper mulberry extract, ethyl ascorbyl ether (oil-soluble licorice extract), and the like.

As the moisturizer, one or more selected from the group consisting of hydroxy proline, glycerin, glycerol, urea, amino acids, lactate, and pyroglutamic acid may be used.

In addition, if necessary, a metal chelating lipid may be further included in the lipid nanoparticle of the present invention. Preferably, one or more selected from the group consisting of DOGS-NTA-Ni lipids (*1,2-dioleoyl-*sn*-glycero-3-[(N-(5-amino-1-carboxypentanyl)aminodiacetic acid)succinyl] (nickel salt)), DMPE-DTPA-Gd lipids (1,2-dimyristoyl-*sn*-glycero-3-phosphoethanolamine-N-diethylenetriaminepentaacetic acid (gadolinium salt), and DMPE-DTPA-Cu lipids (1,2-dimyristoyl-*sn*-glycero-3-phosphoethanolamine-N-diethylenetriaminepentaacetic acid (copper salt)) may be used.

In another sense, the metal chelating lipid may be referred to as an ionizable lipid. The ionizable lipid is an ionizable compound having properties similar to lipids and may serve to allow the drug to be encapsulated in the lipid nanoparticles with high efficiency through electrostatic interaction with the drug (for example, anionic drug and/or nucleic acid). The ionizable lipid may be protonated (positively charged) at a pH below the pKa of the cationic lipid and substantially neutral at a pH above the pKa. In an example, the lipid nanoparticle may comprise a protonated ionizable lipid and/or an ionizable lipid exhibiting neutrality.

Antibodies may be bound to the lipid nanoparticles, if necessary. The antibody is an antibody capable of specifically binding to a receptor or antigen on the cell surface and may improve material delivery efficiency into the nucleus of the target cell. For example, polyclonal antibodies or monoclonal antibodies against biological components such as a cell membrane protein receptor that specifically expresses in a target tissue or organ are preferably disposed on the surface of lipid nanoparticles.

The antibody may be easily prepared using techniques well known in the art. The polyclonal antibody can be obtained from serum collected after injecting an animal with a first-class protein specifically expressed on the surface of a target cell. As the animal, any animal host such as goat, rabbit, or pig may be used. The monoclonal antibody may be prepared using a hybridoma method (Kohler G. and Milstein C.) or a phage antibody library (Clackson et al.; Marks et al.), as is well known in the art to which the present invention pertains. In order to perform the hybridoma method, cells of an immunologically suitable host animal such as a mouse, and cancer or myeloma cell lines may be used. Thereafter, these two types of cells may be fused, and antibody-producing cells may be proliferated using a standard tissue culture method, by a method using polyethylene glycol or the like, that is, a method well known in the art to which the present invention pertains. Subsequently, after a uniform cell population is obtained by subcloning using a limited dilution technique, a hybridoma capable of producing an antibody specific for a type 1 protein specifically expressed on the target cell surface may be mass-cultured *in vitro* or *in vivo* according to a standard technique. The phage antibody library method may be performed by obtaining an antibody gene for a first type protein which is specifically expressed on the surface of a target cell, expressing the antibody gene in the form of a fusion protein on the surface of a phage to prepare an antibody library *in vitro,* and separating and preparing a monoclonal antibody binding to the first type protein which is specifically expressed on the surface of a target cell from the library. The antibodies prepared by the above methods may be separated by electrophoresis, dialysis, ion exchange chromatography, affinity chromatography, etc.

The antibody may comprise a functional fragment of an antibody molecule, as well as a complete form having two full length light chains and two full length heavy chains. The functional fragment of an antibody molecule means a fragment that possesses at least an antigen-binding function, and includes Fab, F(ab'), F(ab')2, F(ab)2, Fv, etc.

The antibody may be bound by using one or more crosslinking agents selected from the group consisting of 1,4-bis-maleimidobutane, 1,11-bis-maleimidotetraethylene glycol, 1-ethyl-3-[3-dimethyl aminopropyl] carbodiimide hydrochloride, succinimidyl-4-[N-maleimidomethylcyclohexane-1-carboxy-[6-amidocaproate] and sulfonates thereof (sulfo-SMCC), succinimidyl 6-[3-(2-pyridyldithio)-ropionamido] hexanoate] and sulfonates thereof (sulfo-SPDP), m-maleimidobenzoyl-N-hydroxysuccinimide ester and sulfonates thereof (sulfo-MBS), succinimidyl[4-(p-maleimidophenyl) butylate] and sulfonates thereof (sulfo-SMPB) as a linker. In this case, the linker is characterized in that it connects the PEG-modified lipid of the lipid nanoparticle to the antibody.

### Effect of the invention

Long-chain ceramides, such as exogenous C18-ceramide, have a very low solubility in an aqueous solution and thus were limited in use, and even when used as a drug, cell delivery properties are low. Accordingly, the present invention provides lipid nanoparticles comprising long-chain ceramide, and a preparation method thereof. According to the present invention, long-chain ceramides such as C18-ceramide can be delivered to *in vivo* targets through lipid nanoparticles, and thus cell death can be effectively induced as desired and the utility as a cell therapy product having high water-solubility and water-dispersibility can be enhanced.

### Brief description of the drawings

FIG. 1 shows a schematic diagram of the structure of lipid nanoparticles comprising C18-ceramide.
FIG. 2 shows ¹H NMR results for C18-ceramide used in the present invention.
FIG. 3 shows DLS data results of lipid nanoparticles prepared except for C18-ceramide (a:Number, b:lntensity).
FIG. 4 shows DLS data results of lipid nanoparticles including 0.1mM C18-ceramide (a:Number, b:lntensity).

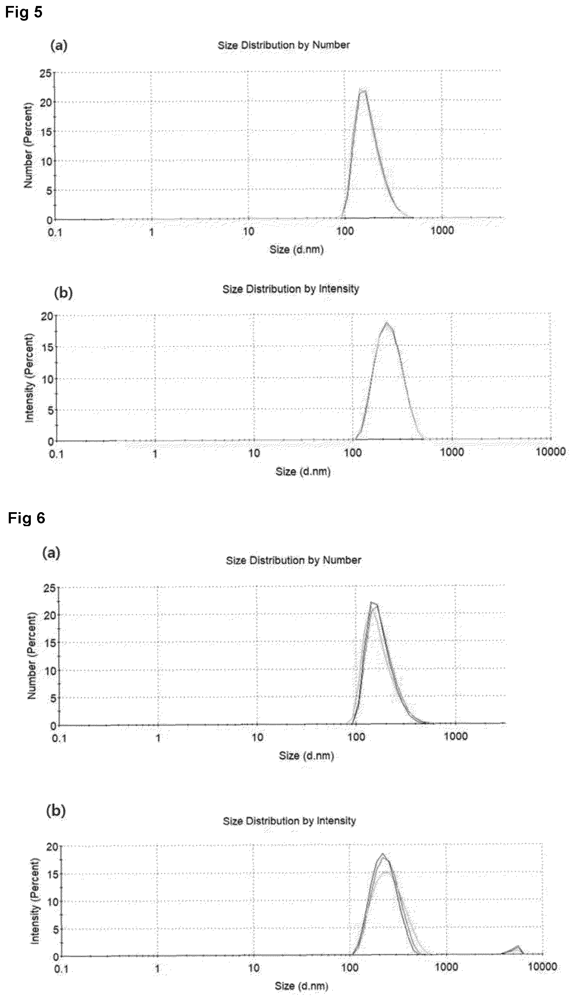

FIG. 5 shows DLS data results of lipid nanoparticles including 0.5mM C18-ceramide (a:Number, b:lntensity).
FIG. 6 shows DLS data results of lipid nanoparticles including 1mM C18-ceramide (a:Number, b:lntensity).

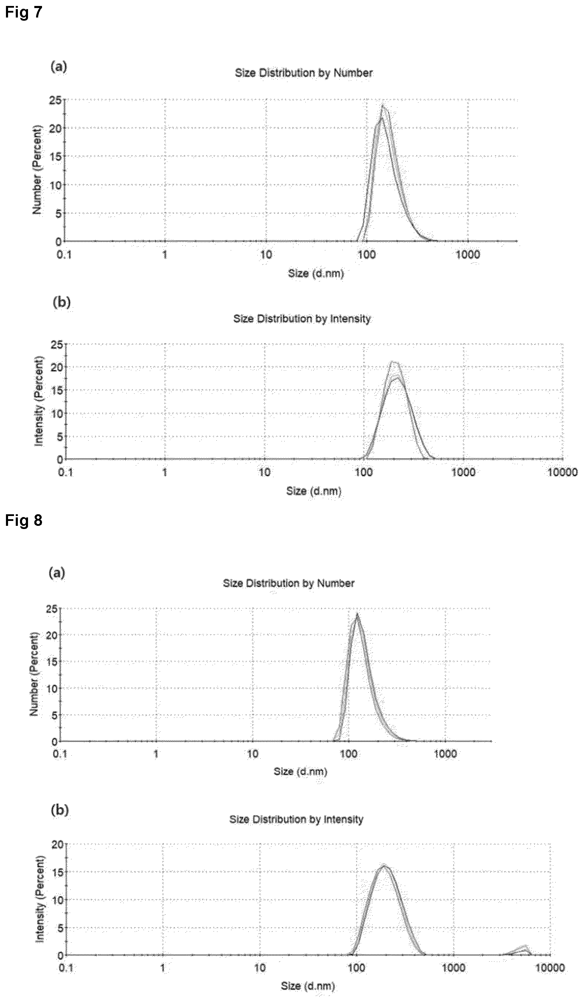

FIG. 7 shows DLS data results of lipid nanoparticles including 2mM C18-ceramide (a:Number, b:lntensity).
FIG. 8 shows DLS data results of lipid nanoparticles including 5mM C18-ceramide (a:Number, b:lntensity).
FIG. 9 shows the results of confirming the survival rate of cells by treating MDA-MB-231 cells and MDA-MB-468 cells with C18-ceramide-containing lipid nanoparticles (a:MDA-MB-231, b:MDA-MB-468).
FIG. 10 shows the results of confirming the expression of apoptosis-related proteins through Western blotting by treating MDA-MB-468 cells with lipid nanoparticles containing C18-ceramide.

### Form for practice of the invention

Hereinafter, preferred embodiments of the present invention will be described in detail. However, the present invention is not limited to the embodiments described herein and may be embodied in other forms. Rather, it is provided to fully convey the spirit of the present invention to those skilled in the art so that the content introduced here is thorough and complete.

Example 1. Synthesis of Ceramide-Containing Lipid Nanoparticles (LNPs) Using Thin Film Hydration Method

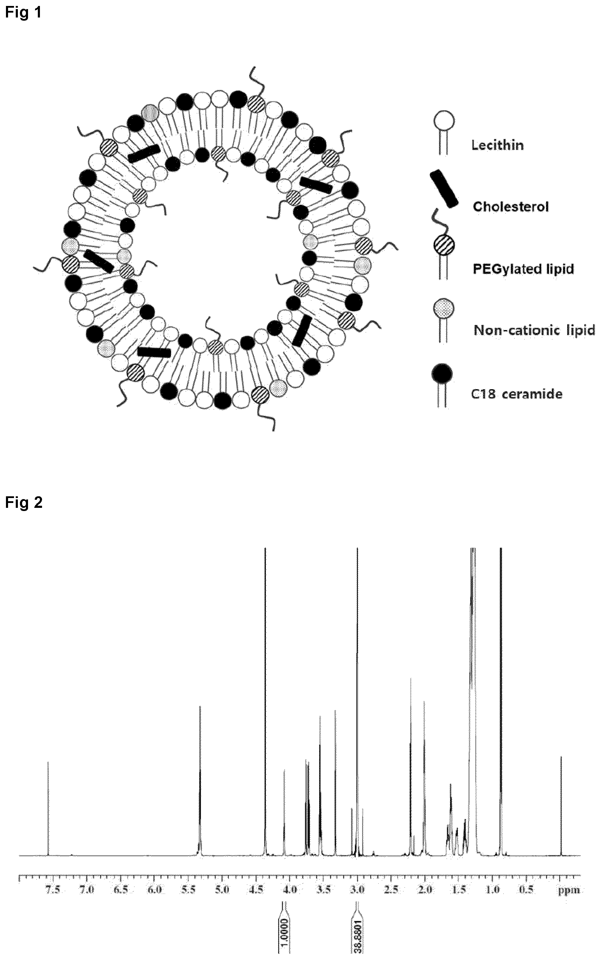

Five different lipids were added to 1 mL of chloroform at the following concentrations:
1 mM lecithin;
0.2 mM cholesterol;
0.05 mM DSPC;
0.1mM DSPE-PEG(2000) Amine; and;
C18-ceramide (N-Oleoyl-phytosphingosine, Chemical Formula 2) - 0, 0.1, 0.5, 1, 2, 5 mM by concentration. (C18-ceramide has a purity of 95.3%, and ¹H NMR results are shown in FIG. 2)

After that, when each raw material was dissolved, the chloroform solvent was evaporated to form a lipid film. Next, the lipid film was hydrated with 1 mL phosphatebuffered saline (PBS), pH 7.2. After the hydrated liquid phase was subjected to sonication, a freeze-thaw procedure of repeating the procedure of freezing and thawing the hydrated lipid film using liquid nitrogen five times was performed, and then the hydrated liquid phase was subjected to sonication again to obtain a liquid phase in a dispersed state, and then the LNP dispersed solution was purified with Sepharose CL-4B size extraction column and filtered through a 0.2 µm syringe filter to finally obtain lipid nanoparticles (LNPs).

### Example 2. Synthesis of Ceramide-Containing Lipid Nanoparticles (LNPs) Using Microfluics Method

Five different lipids were added to 1 mL of ethanol at the following concentrations:
1 mM lecithin;
0.2 mM cholesterol;
0.05 mM DSPC;
0.1mM DSPE-PEG(2000) Amine; and;
C18-ceramide (N-Oleoyl-phytosphingosine, Chemical Formula 2) - 0, 0.1, 0.5, 1, 2, 5 mM by concentration.

Thereafter, when each raw material was dissolved, the solution was flowed through an organic solvent line of a microfluidics instrument, which is a droplet-based microfluidic system, in order to prepare a dispersion of lipid nanoparticles, and water was flowed through a water solvent line. In this case, the solvent was mixed in a volume ratio of ethanol:water of 1:3. After obtaining a liquid phase in a dispersion state in which lipid nanoparticles were formed, the LNP dispersion was purified with Sepharose CL-4B size extraction column, and then filtered through a 0.2 µm syringe filter to finally obtain lipid nanoparticles (LNPs). Finally, ethanol was removed using a solvent substituent.

### Example 3. Characterization of Lipid Nanoparticles (LNPs) Containing C18-Ceramide

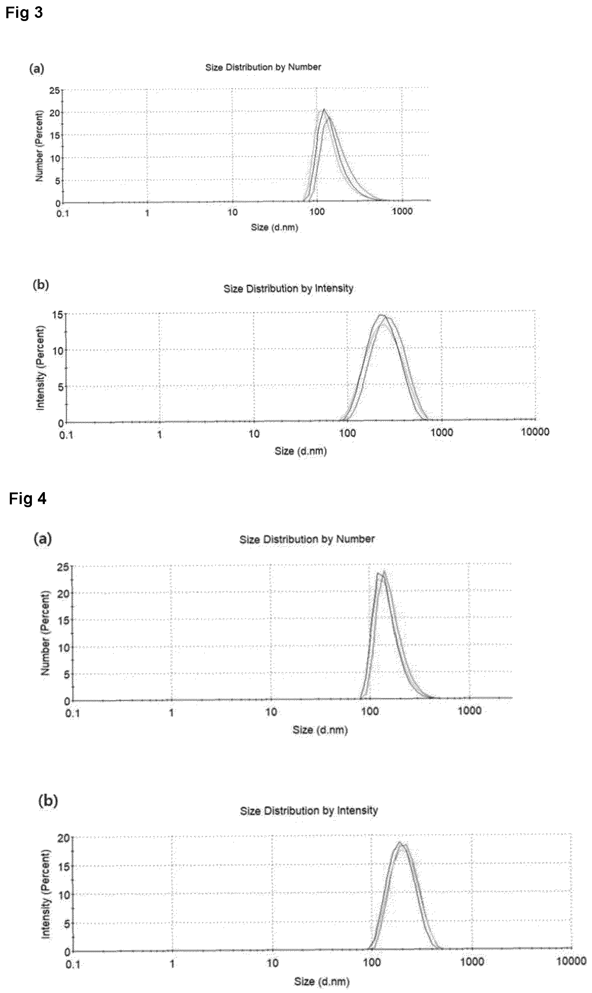

The lipid nanoparticles prepared for each concentration of C18-ceramide were analyzed by dynamic light scattering (DLS) analysis and zeta potential measurement to analyze dispersibility and surface charge, and the results are shown in Table 1 and FIG. 3. In this case, since the results of the lipid nanoparticles obtained in Example 1 and Example 2 were almost similar to each other, all the subsequent results were performed for each sample, but the results of the lipid nanoparticles obtained in Example 2 were presented as representative.

**[Table 1]**

| C18-Ceramide concentration of NPs | DLS data (PDI) | Zeta-potential (mV) |
|---|---|---|
| 0 mM | 0.13 | -13.2 ± 0.93 |
| 0.1 mM | 0.16 | -11.8 ± 1.05 |
| 0.5 mM | 0.15 | -11.15 ± 1.08 |
| 1 mM | 0.19 | -11.1 ± 0.75 |
| 2 mM | 0.16 | -10.54 ± 0.94 |
| 5 mM | 0.19 | -11.73 ±2.16 |

According to the results of Table 1 and FIG. 3, it can be confirmed that even when the concentration of C18-ceramide contained in the lipid nanoparticles is high, the dispersibility of the nanoparticles (PDI; polydispersity index) is all 0.2 or less, so that the dispersibility in water is very excellent.

In addition, particle size and particle concentration were evaluated by NTA (nanoparticle tracking analysis), and the results are shown in Table 2.

**[Table 2]**

| C18-Ceramide co ncentration of NPs | NTA data | |
|---|---|---|
| | Particle size (nm) | Particle concentration (particles/ml) |
| 0 mM | 109.1 ± 9.2 | 3.55 x 10¹² ± 9.56 x 10¹⁰ |
| 0.1 mM | 123.8 ± 0.3 | 5.05 x 10¹²± 2.21 x 10¹¹ |
| 0.5 mM | 155.0 ± 7.4 | 5.83 x 10¹² ± 2.74 x 10¹¹ |
| 1 mM | 160.7 ± 26.1 | 5.19 x 10² ± 1.59 x 10¹¹ |
| 2 mM | 130.1 ± 12.0 | 2.94 x 10¹² ± 4.32 x 10¹¹ |

Referring to the results of Table 2, it was confirmed that all of the lipid nanoparticles prepared for each concentration of C18-ceramide were around 100-150 nm, and the number of particles was synthesized to 10¹² particles/mL or more.

### Example 4. Confirmation of Cell Survival Rate Due to Treatment with Lipid Nanoparticles (LNP) Containing C18-Ceramide

MDA-MB-231 and MDA-MB-468 cells were cultured at 37°C under 10% fetal bovine serum (FBS) and ATCC modified RPMI medium (Gibco) supplemented with 1% penicillin/streptomycin, 5% CO₂.

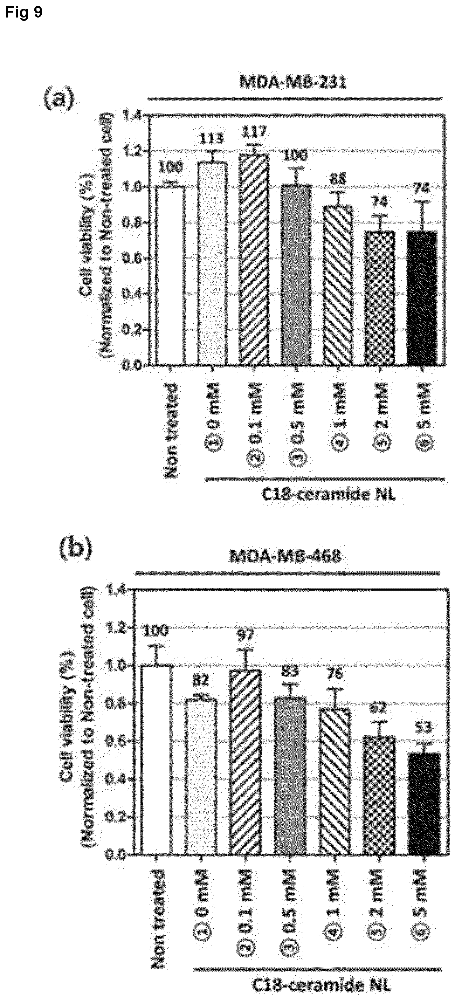

For experiments, lipid nanoparticles prepared at each concentration of C18-ceramide were respectively treated (the number of particles: 4.5 x 10⁸ ± 2.05 x 10⁸) to MDA-MB-231 and MDA-MB-468 cells which were inoculated into a 96-well plate (1.5 x 10³ cells/well). After 24 hours of incubation, WST-8 reagent (Dojindo) was added to the medium at 37°C for 1 hour. Absorbance was measured at 450nm using a microplate reader (BioTek). Thereafter, cell viability was confirmed by measuring absorbance every 24 hours. MDA-MB-231 cells had an average cell viability decrease of 74% when lipid nanoparticles prepared at concentrations of 2 mM and 5 mM C18-ceramide were treated (FIG. 9a).

In addition, the cell viability of MDA-MB-468 cells was reduced to 53% when lipid nanoparticles of 5 mM C18-ceramide were treated (FIG. 9b).

### Example 5. Confirmation of Expression of Antitumor-Related Proteins by Lipid Nanoparticles (LNPs) Containing C18-Ceramide

MDA-MB-468 cells were cultured in ATCC modified RPMI medium (Gibco) supplemented with 10% fetal bovine serum (FBS) and 1% penicillin/streptomycin at 37°C under 5% CO₂.

For experiments, MDA-MB-468 cells were seeded onto 60 mm² cell culture plates (1.3 x 10⁶ cells/plate). After 24 hours, LNP prepared at different concentrations of C18-ceramide was calculated and added to 2.5 mL opti-MEM medium per well such that 2.48 x 10¹¹±2.75 x 10¹⁰ particles were included in the medium. The medium containing LNP was treated and incubated at 37°C for 24 hours.

Thereafter, the cells were collected, and western blotting was performed. For this purpose, MDA-MB-468 cells treated with LNP were lysed in RIPA buffer containing a protease inhibitor and a phosphatase inhibitor (Sigma). Total cell lysates were quantified using the BCA protein assay kit (Thermo Scientific). Next, the cell lysate (50 µg) was loaded on a 4-15% gradient SDS-PAGE gel (Biorad) under denaturing conditions and transferred to a PVDF membrane (iBlot2 Dry Blotting System, Thermo Scientific). The membrane was blocked with 5% skim milk at room temperature for 1 hour, and primary antibodies of BAX, BAD, BCL2, Actin (Santa Cruz), and Cleaved caspase3 (cell signaling) were reacted at 4°C overnight.

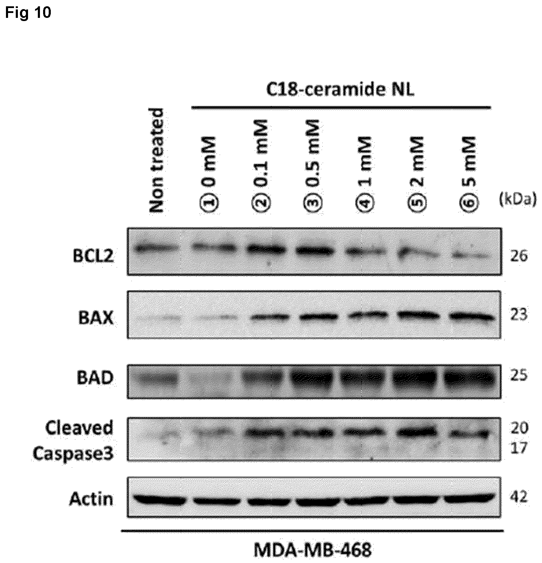

The secondary antibody conjugated with HRP was reacted at room temperature for 2 hours, and then the membrane was treated with Western ECL, and the luminescence image was analyzed using LAS500 (GE Healthcare), and the results are shown in FIG. 10.

As a result of confirming the expression levels of the apoptosis-related proteins (BAX, BAD, BCL2, and cleaved caspase3) through FIG. 10, when lipid nanoparticles including a high concentration of C18-ceramide were treated, the expression level of the anti-apoptosis protein BCL2 tended to decrease, but the expression levels of the pro-apoptosis proteins BAX and BAD tended to increase. In addition, the cleavage form of caspase3, the lowest step of apoptosis signal, was identified, demonstrating that apoptosis was induced in cells by C18-ceramide.

Accordingly, it can be seen that LNP containing C18-ceramide has an excellent killing effect of cancer cells.

In addition, compared to the ceramide-containing LNP, the ceramide alone was dispersed in the aqueous solution in the same amount as in Examples 1 and 2 and subjected to column purification to perform the cell survival rate of Example 4 and the antitumor-related protein expression experiment of Example 5, but it was confirmed that there was no change. Since the C18-ceramide itself was insoluble in an aqueous solution, it was found that the C18-ceramide itself did not exhibit the effect of the C18-ceramide itself because the C18-ceramide as an active ingredient was not present in the aqueous solution during the cell viability and anti-tumor protein expression test.

## Claims

1. A lipid nanoparticle comprising a long-chain ceramide represented by the following Chemical Formula 1. 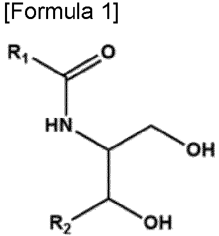 In Chemical Formula 1, R₁ is a saturated or unsaturated aliphatic chain having 17 carbon atoms, R₂ is one selected from phytosphingosine, sphingosine, and sphinganine, and the number of carbon atoms is 10 to 16.

2. A lipid nanoparticle of claim 1 **characterized in that** the long-chain ceramide having the structure of the above Chemical Formula 1 is a compound selected from an N-oleoyl-phytosphingosine-based compound or a derivative thereof; a N-stearoyl-erythro-sphingosine-based compound or a derivative thereof; a N-oleoyl-erythro-sphingosine-based compound or a derivative thereof; a N-stearoyl-erythro-sphinganine-based compound or a derivative thereof; and a N-oleoyl-erythro-sphinganine-based compound or a derivative thereof.

3. A lipid nanoparticle of claim 1 **characterized in that** the lipid nanoparticle comprises at least one lipid selected from lecithin, cholesterol, non-cationic lipids, and polyethyleneglycol (PEG)-modified lipids.

4. A lipid nanoparticle of claim 3 **characterized in that** the non-cationic lipid is a lipid comprising at least one selected from a neutral lipid and an anionic lipid.

5. A lipid nanoparticle of claim 4 **characterized in that** the neutral lipid is at least one selected from a group consisting of HSPC (hydrogenated soy phosphatidylcholine), DOPC (1, 2-dioleoyl-*sn*-glycero-3-phosphocholine), POPE (1-palmitoyl-2-oleoyl-*sn*-glycero-3-phosphoethanolamine), DSPC (1, 2-distearoyl-*sn*-glycero-3-phosphocholine), DSPE (1, 2-distearoyl-*sn*-glycero-3-phosphoethanolamine), DPPC (1, 2-dipalmitoyl-*sn*-glycero-3-phosphocholine), POPC (1-palmitoyl-2-oleoyl-*sn*-glycero-3-phosphocholine), DOPE (1, 2-dioleoyl-*sn*-glycero-3-phosphoethanolamine), SM (N-palmitoyl-D-erythrospinolphosphorylcholine), DLPE (1, 2-dilauroyl-*sn*-glycero-3-phosphoethanolamine), DPPE (1, 2-diphytanoyl-*sn*-glycero-3-phosphoethanolamine), phosphatidylcholine, dimyristoylglycerol, succinoyl-diacylglycerol, phosphatidyl ethanolamine, tetraether lipid, sphingolipid, diacryl glycerol and glyceride, or a combination thereof.

6. A lipid nanoparticle of claim 4 **characterized in that** the anionic lipid may be at least one selected from DOPS(dioleoylphosphatidylserine), DMPG(dimyristoylphosphatidylglycerol), DPPG(dipalmitoylphosphatidylglycerol), DTPA(diethylenetriamine pentaacetic acid), DPTGA(1,4-dipalmitoyl-tartarate-2,3-diglutaric acid), DSTSA(1,4-disteroyl-tartarate-2,3-disuccinic acid), CHHDA(2-carboxyheptadecanoyl heptadecylamide), DMPS(dimyristoylphosphatidylserine), DPPS(dipalmitoylphosphatidylserine), POPS(palmitoyloleoylphosphatidylserine), DOPG(dioleoylphosphatidylglycerol), POPG(palmitoyloleoylphosphatidylglycerol), DMPA(dimyristoylphosphatidic acid), DPPA(dipalmitoylphosphatidic acid), DOPA(dioleoylphosphatidic acid), POPA(palmitoyloleoylphosphatidic acid), CetylP(Cetyl phosphate), CHEMS(cholesterol hemisuccinate), or a combination thereof.

7. A lipid nanoparticle of claim 3 **characterized in that** the PEG-modified lipid is a combination of a non-cationic lipid and polyethylene glycol (PEG).

8. A lipid nanoparticle of claim 3 **characterized in that** the lipid nanoparticles have a particle size of 10 to 2,000 nm.

9. A nanoparticle delivery composition for cell apoptosis, comprising the lipid nanoparticles according to any one of claims 1 to 8.

10. The nanoparticle delivery composition for cell apoptosis according to claim 9, wherein the cells are cancer cells.

11. The nanoparticle delivery composition for cell apoptosis, wherein the cancer cell is derived from a cancer tissue selected from angiomas, vascular fibromas, lung cancer, non-small cell lung cancer, liver cancer, colon cancer, bone cancer, pancreatic cancer, skin cancer, head cancer, cervical cancer, melanoma, uterine cancer, ovarian cancer, rectal cancer, stomach cancer, anal cancer, colon cancer, breast cancer, fallopian tube carcinoma, endometrial carcinoma, cervical carcinoma, vaginal carcinoma, vulvar carcinoma, Hodgkin's disease, oesophageal cancer, small intestine cancer, endocrine gland cancer, thyroid cancer, parathyroid cancer, adrenal cancer, soft tissue sarcoma, urethral cancer, penile cancer, prostate cancer, bladder cancer, kidney or ureter cancer, kidney cell carcinoma, kidney pelvic carcinoma, central nervous system tumor, primary central nervous system lymphoma, spinal cord tumor, brainstem glioma, and pituitary adenoma.

12. An anticancer pharmaceutical composition comprising the nanoparticle delivery composition of claim 9.

13. A method for preparing lipid nanoparticles, comprising:
(Step 1) preparing a mixed composition by mixing one lipid selected from lecithin, cholesterol, a non-cationic lipid, and a polyethylene glycol (PEG)-modified lipid; and a long-chain ceramide having a structure of the following Chemical Formula 1 in an organic solvent;
(Step 2) preparing the mixed composition in a dispersion state and column purifying; and
(Step 4) sterile-filtering a purified product obtained by column purification; 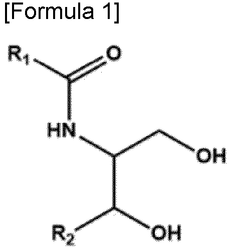 In Chemical Formula 1, R₁ is a saturated or unsaturated aliphatic chain having 17 carbon atoms, R₂ is one selected from phytosphingosine, sphingosine, and sphinganine, and the number of carbon atoms is 10 to 16.

14. The method for preparing a lipid nanoparticle of claim 13, wherein the long-chain ceramide having the structure of Chemical Formula 1 is a compound selected from a N-oleoyl-phytosphingosine-based compound or a derivative thereof; a N-stearoyl-erythro-sphingosine-based compound or a derivative thereof; a N-oleoyl-erythro-sphingosine-based compound or a derivative thereof; a N-stearoyl-erythro-sphingosine-based compound or a derivative thereof; a N-stearoyl-erythro-sphinganine-based compound or a derivative thereof; and N-oleoyl-erythro-sphinganine-based compounds or derivatives thereof.

15. The method for preparing the lipid nanoparticles of claim 13, wherein the non-cationic lipid is a lipid comprising any one or more selected from a neutral lipid and an anionic lipid.

16. The method for preparing the lipid nanoparticles of claim 15, wherein the neutral lipid is at least one selected from a group consisting of HSPC (hydrogenated soy phosphatidylcholine), DOPC (1, 2-dioleoyl-*sn*-glycero-3-phosphocholine), POPE (1-palmitoyl-2-oleoyl-*sn*-glycero-3-phosphoethanolamine), DSPC (1, 2-distearoyl-*sn*-glycero-3-phosphocholine), DSPE (1, 2-distearoyl-*sn*-glycero-3-phosphoethanolamine), DPPC (1, 2-dipalmitoyl-*sn*-glycero-3-phosphocholine), POPC (1-palmitoyl-2-oleoyl-*sn*-glycero-3-phosphocholine), DOPE (1, 2-dioleoyl-*sn*-glycero-3-phosphoethanolamine), SM (N-palmitoyl-D-erythro-spinolphosphorylcholine), DLPE (1, 2-dilauroyl-*sn*-glycero-3-phosphoethanolamine), DPPE (1, 2-diphytanoyl-*sn*-glycero-3-phosphoethanolamine), phosphatidylcholine, dimyristoylglycerol, succinoyl-diacylglycerol, phosphatidyl ethanolamine, tetraether lipid, sphingolipid, diacryl glycerol and glyceride, or a combination thereof.

17. The method for preparing the lipid nanoparticles of claim 15, wherein the anionic lipid may be at least one selected from DOPS(dioleoylphosphatidylserine), DMPG(dimyristoylphosphatidylglycerol), DPPG(dipalmitoylphosphatidylglycerol), DTPA(diethylenetriamine pentaacetic acid), DPTGA(1,4-dipalmitoyl-tartarate-2,3-diglutaric acid), DSTSA(1,4-disteroyl-tartarate-2,3-disuccinic acid), CHHDA(2-carboxyheptadecanoyl heptadecylamide), DMPS(dimyristoylphosphatidylserine), DPPS(dipalmitoylphosphatidylserine), POPS(palmitoyloleoylphosphatidylserine), DOPG(dioleoylphosphatidylglycerol), POPG(palmitoyloleoylphosphatidylglycerol), DMPA(dimyristoylphosphatidic acid), DPPA(dipalmitoylphosphatidic acid), DOPA(dioleoylphosphatidic acid), POPA(palmitoyl-oleoylphosphatidic acid), CetylP(Cetyl phosphate), CHEMS(cholesterol hemisuccinate), or a combination thereof.

18. The method for preparing the lipid nanoparticles of claim 13, wherein the PEG-modified lipid is a combination of a non-cationic lipid and polyethylene glycol (PEG).

19. The method for preparing the lipid nanoparticles of claim 13, wherein the organic solvent may be selected from methanol, ethanol, benzene, butanol, butyl acetate, carbon tetrachloride, chloroform, cyclohexane, dichloroethane, dichloromethane, diethyl ether, diisopropyl ether, ethyl acetate, heptane, hexane, isooctane, methyl ethyl ketone (MEK), methyl t-butyl ether (MTBE), pentane, toluene, trichloroethylene, xylene, and mixed solvents thereof.

20. The method for preparing lipid nanoparticles of claim 13, **characterized in that** a dispersion is prepared by sonication or freeze-thawing in the step 2.

21. The method for preparing lipid nanoparticles of claim 13, **characterized in that** in the step 2, a dispersion is prepared using a droplet-based microfluidic system.

22. The method for preparing lipid nanoparticles of claim 13, **characterized in that** the filter used in the sterile filtration of the step 3 has a pore size of 0.25 to 0.4 µm.
